# EUROPEAN PATENT SPECIFICATION

(11) **EP 3 057 639 B1**
(45) Date of publication and mention of the grant of the patent: **27.03.2019**
(21) Application number: 14853279.9
(22) Date of filing: 15.10.2014
(51) Int. Cl.: A61M 16/20, F16K 11/00, A61M 16/00, A61M 16/04, A61M 16/06, A61M 16/08, A61M 16/10

(54) **IMPROVED OXYGENATING APPARATUS**
VERBESSERTE SAUERSTOFFANREICHERUNGSVORRICHTUNG
APPAREIL AMÉLIORÉ D'OXYGÉNATION

(30) Priority: 15.10.2013 AU 2013903968
(43) Date of publication of application: 24.08.2016
(73) Proprietor: Wallis Coombe Pty Ltd as Trustee for Sleuky Family Trust, Launceston, Tasmania 7250 (AU)
(72) Inventor: BATTERSBY, Scott, Lonsdale DC, South Australia 5160 (AU); WALLIS, Andrew, East Launceston, Tasmania 7250 (AU)
(74) Representative: Burrows, Anthony Gregory
(86) International application number: PCT/AU2014/050288
(87) International publication number: WO 2015/054747

(56) References cited:
- WO-A1-97/10018
- WO-A2-2006/091829
- WO-A2-2006/119191
- GB-A- 2 162 070
- US-A1- 2004 069 308
- US-A1- 2010 319 691
- US-A1- 2012 048 274
- US-A1- 2012 048 274

## Description

### FIELD OF THE INVENTION

The present invention relates to a disposable oxygenating apparatus and in particular to a single use disposable positive pressure oxygenating apparatus for assisting in a patient's recovery after anaesthesia or a patient otherwise requiring respiratory support.

### BACKGROUND OF THE INVENTION

When patients are waking from anaesthesia they typically have an endotracheal tube (ETT) or laryngeal mask airway (LMA) *in situ,* and their breathing is usually relatively slow and shallow. This means that they are under-ventilating their lungs and are at risk of desaturation, the condition of low blood oxygen concentration. Medical personnel combat this problem by increasing the patient's fraction of inspired oxygen (FiO₂). This can be achieved by attaching an oxygenation device to the end of the endotracheal tube or laryngeal mask airway.

An existing device disclosed in WO1997/010018 and sold under the T-Bag trade mark, comprises a 'T-shaped' pipe having an open outlet, an inlet connected to a collapsible reservoir bag and a coupling for attachment to an end of the ETT or LMA. Oxygen is delivered into the collapsible reservoir bag and the oxygen is entrained into the inflow of air as the patient breathes in. However because the oxygen is only entrained into the inflow of air, and the patient's breathing may only be shallow, the fraction of inspired oxygen may not be very high. The effectiveness of this currently used device is therefore limited and there is still the significant risk of desaturation occurring.

US2012048274 discloses a respiratory valve apparatus with a housing having an inner chamber, an endotracheal tube connection port, a respirator connection port and a resuscitation bag connection port. A valve positioned within the inner chamber can switch the flow between a manual resuscitation bag port and a ventilator port enabling the patient to be treated without having to disconnect the respirator support system to thereby connect the resuscitation bag. This prevents the loss of positive end expiratory pressure (PEEP) in the lungs and guards against lung collapse and hemodynamic compromise. The valve includes preloaded seals that will create minimal dragging during valve actuation and work under both positive and negative pressure. The apparatus includes a tethered cover for closure of the resuscitation bag port for sealably covering the port when a bag is not attached or the ventilator connector during patient transport. A sealing arrangement within the resuscitator bag port insures that PEEP in maintained when the resuscitator bag adapter is inserted into the housing.

Furthermore, as the patient wakes from anaesthesia the volume of each inspired breath increases. The patient's respiratory rate increases from around 8-12 breaths per minute to around 20-30 breaths per minute, and the minute volume increases from around 2 litres (L) to around 9 litres (L), or more. Additionally, as the patient awakes they may be in acute pain, which means their respiratory rate and heart rate both significantly increase. The collapsible reservoir bag disclosed in the above prior art, is however relatively small and can be quickly emptied during inspiration, under such circumstances.

Other medical issues occur because the vocal cords of patients with a LMA *in situ* can close in the time between when a patient is anaesthetised and when they are fully awake. During this waking process the vocal cords are hyper-reactive and they may go into spasm when foreign matter (eg. saliva, sputum, blood etc) come into contact with them. At this time the patient may not be sufficiently awake to clear the foreign matter. The full or partial closing of the vocal cords can lead to respiratory difficulty, desaturation and negative pressure pulmonary edema. Although positive end expiratory pressure (PEEP) or continuous positive airway pressure (CPAP) can be used to increase the pressure within the pharynx to maintain the vocal cords open, none of the existing disposable oxygenating devices have the capacity for CPAP or PEEP,

Similar desaturation issues may occur with patients that have other medical conditions, such as but not limited to, heart failure or chronic obstructive pulmonary disease, and accordingly requiring respiratory support. Furthermore many respiratory support devices are costly.

It should be appreciated that any discussion of the prior art throughout the specification is included solely for the purpose of providing a context for the present invention and should in no way be considered as an admission that such prior art was widely known or formed part of the common general knowledge in the field as it existed before the priority date of the application.

### SUMMARY OF THE INVENTION

The invention is defined by the appended claims.

In one aspect of the invention, but not necessarily the only or broadest aspect, there is proposed a disposable oxygenating apparatus for use on a patient recovering from anaesthesia or otherwise requiring respiratory support, where a respiratory device is in situ or is to be used, including,
a body portion having a coupling for attachment to said respiratory device,, a passageway or passageways for fluid communication with the respiratory device, the passageway or passageways extending through said body portion between said coupling and both a primary inlet and a secondary inlet, and extending through said body portion between said coupling and an outlet, for fluid communication therebetween,
a collapsible reservoir bag attached over said primary inlet, said collapsible reservoir bag in fluid communication with a source of oxygen or oxygen rich air,
an expiratory valve or valves located in or adjacent said outlet for controlling the passage of an expired breath from said patient during expiration, wherein a positive pressure is applied to the airway of said patient during expiration and rest, a primary inspiratory valve located in or adjacent said primary inlet, for controlling said oxygen or oxygen rich air flowing in through said passageway or passageways from said collapsible reservoir bag, for at least a period of time during inspiration by said patient, and
a secondary inspiratory valve located in or adjacent said secondary inlet in fluid communication with said passageway or passageways for controlling entry of an ambient air in through the passageway or passageways from an exterior of said apparatus during inspiration when said collapsible reservoir bag has been substantially emptied.

The respiratory device may be an endotracheal tube or laryngeal mask airway, wherein the apparatus is attached at an end of an endotracheal tube or a tube of said laryngeal mask airway, which extends outwardly from an airway of said patient. In another form the respiratory device includes a face mask that is attached over said patient's mouth and nose, wherein the apparatus is attached to said face mask or a tube connected to said face mask. In still another form the respiratory device may be a nasal airway respiratory support device.

It should be appreciated that the phrase respiratory device includes all types of invasive and non-invasive respiratory support devices, including but not limited to, Laryngeal Mask Airway (LMA), and Endotracheal Tube (ETT).

The apparatus is preferably a single use disposable apparatus.

The apparatus may be used on non-invasive positive pressure devices having a face mask to help the patient breath, as a result of heart failure, chronic obstructive pulmonary disease, etc.

In one form the expiratory valve, located in or adjacent said outlet, is an in-line check valve. The in-line check valve may include a disc attached to a shaft and a helical spring positioned around the shaft for biasing the disc against a valve seat.

Preferably the expiratory valve opens at low pressure to inhibit over pressurisation of the patient's airway. The expiratory valve preferably maintains a positive end expiratory pressure, wherein the pressure within the airway of the patient is between 4-14 centimetres of water (cmH₂O) and more preferably 8-12 centimetres of water (cmH₂O).

The expiratory valve is opened at a low pressure and can rapidly dissipate the expired air in the event of a sudden increase in the rate of expiration, being the exhalation of breath from the lungs, or a peak in expiration volume and/or pressure, such as when the patient coughs.

The expiratory valve may be a biased check valve, swing check valve or tilting disc check valve.

The primary and secondary inlets may be spaced apart from each other at different locations along a single passageway, or along different passageways. Alternatively, the primary and secondary inlets may be located at the same location along the single passageway. In one form the primary and secondary inlets may be coaxially aligned in that the primary inlet is centrally located within the secondary inlet and separated from the secondary inlet by an annular, or otherwise shaped, wall. The collapsible reservoir bag being secured to said wall such that it covers the primary inlet.

The primary inspiratory valve may be a diaphragm check valve and in one form is an umbrella valve. The diaphragm check valve includes a flexible rubber diaphragm positioned over at least one aperture in the primary inlet. At rest the flexible rubber diaphragm seals the at least one aperture in the primary inlet. In another form the primary inlet includes a plurality of apertures. The flexible rubber diaphragm in one form is a disc that is secured at a central location to a mount, the mount including or forming said at least one aperture. Preferably the flexible rubber diaphragm is resiliently flexible around at least its edge.

The secondary inspiratory valve may be a diaphragm valve and in one form is an umbrella valve.

The primary inspiratory valve preferentially opens before the secondary inspiratory valve, In this way the oxygen or oxygen rich air in the collapsible reservoir bag is inhaled first, and once the collapsible reservoir bag is substantially emptied, the secondary inspiratory valve opens to provide the patient with a source of breathable air to inhibit injury to the patient The primary inspiratory valve preferably has a lower opening pressure compared to the secondary inspiratory valve so that it is preferentially opened before the secondary inspiratory valve.

The primary and secondary inspiratory valves may be recessed into the side of the passageway or passageways to inhibit saliva, condensation, or blood fouling the valves during use. Although the addressee should appreciate that the valves may not be recessed and a filter or filters may be used to inhibit saliva, condensation, or blood fouling the valves during use.

In another form the expiratory valve and the secondary inspiratory valve are a combination valve or are formed in a unitary device that comprises an expiratory outlet valve portion and an ambient air inspiratory inlet valve portion.

The combination valve may comprise an expiratory outlet valve of a diaphragm umbrella valve type configuration and an ambient air inspiratory inlet valve of a duckbill valve type configuration, having an end that extends into the passageway or passageways.

In another form a combination primary and secondary inspiratory valve or a two-stage inspiratory valve, can be used. The combination or two-stage inspiratory valve may be located within a casing having a frame including central apertures extending therethrough, in fluid communication with the collapsible reservoir bag, and peripheral apertures in fluid communication with the ambient air. A disc shaped flexible diaphragm may be attached to the frame and configured to reversibly seal the central apertures, and the ring-shaped flexible diaphragm configured to reversibly seal the peripheral apertures.

The two-stage inspiratory valve is configured such that the disc shaped flexible diaphragm opens first, and air is preferentially drawn from within the collapsible reservoir bag, during inspiration. In the event that the collapsible reservoir bag is substantially emptied the ring-shaped flexible diaphragm moves to open peripheral apertures to allow ambient air to be drawn in from the exterior of the apparatus.

The disc shaped flexible diaphragm may be located centrally of the ring-shaped flexible diaphragm wherein the disc shaped flexible diaphragm is attached to a central protrusion and the ring-shaped flexible diaphragm is attached to the frame around an inner edge such that an outer portion of the ring-shaped flexible diaphragm is permitted to flex.

Alternatively, the ring-shaped flexible diaphragm may include a central webbing that is used to attach the ring-shaped flexible diaphragm to the central protrusion and the ring-shaped flexible diaphragm is configured to flex around an outer edge. In this form the disc shaped flexible diaphragm is located on the downstream side of the ring-shaped flexible diaphragm and overlays the webbing.

In still another form the expiratory valve could be understood to comprise a two-stage expiratory valve or cooperating first and second expiratory valves. The expiratory valve comprises a central flexible portion or first expiratory valve, and a larger outer donut shaped flexible diaphragm portion or second expiratory valve. The central flexible portion or first expiratory valve may be disc shaped and be attached to a central projection or may include a plurality of cooperating leaflets.

The two-stage expiratory valve is configured such that the central flexible portion or first expiratory valve opens first, and air is preferentially expelled therethrough, from within the passageway or passageways during expiration In the event that the patient coughs the outer donut shaped flexible diaphragm portion or second expiratory valve is then configured to open to thereby rapidly dissipate the expired air.

Accordingly the central flexible portion or first valve assists in maintaining a positive airway pressure during normal expiration of around 8 cmH₂O, while the larger outer donut shaped flexible diaphragm portion or second expiratory valve, controls the pressure during high flow or high pressure events, such as when the patient coughs, to thereby inhibit injury to the patient or dislodgement of the oxygenation apparatus, ETT or LMA.

The two-stage expiratory valve or cooperating first and second expiratory valves may comprise a valve frame including an outer ring-shaped valve seat, connected to an inner ring-shaped valve seat by radially extending arms. The outer ring-shaped valve seat may be 5cm in diameter and the inner ring-shaped valve seat may be 2cm in diameter. The skilled addressee will appreciate that other types of valves and differently shaped valves or valve seats may be used without departing from the scope of the invention.

In one form the valve or valves may be check valve assemblies that permit control of fluid flow (liquid or gas) along a flow path. A check valve or one-way valve allows fluid flow in a single direction while inhibiting backflow. The check valve may comprise a diaphragm valve or may include a disc-shaped head that is biased against a valve seat, wherein a guide controls movement of the disc-shaped head so that it can reseat properly when the pressure on the upstream side reduces, thereby preventing reverse fluid flow. Accordingly the expiratory valve, primary inspiratory valve and secondary inspiratory valve may each be a diaphragm check valve, ball check valve, duckbill valve, tilting disc check valve, lift-check valve or in-line check valve of similar configuration or different configurations.

A plurality of cooperating leaflets may be connected to the inner ring-shaped valve seat and extend inwardly thereof to form the central flexible portion or first expiratory valve. For discussion purposes the plurality of cooperating leaflets may open at about 12 cmH₂O and closes at about 8 cmH₂O.

The outer donut shaped flexible diaphragm portion or second expiratory valve may have a larger surface area than the central flexible portion or first expiratory valve. The outer donut shaped flexible diaphragm portion or second expiratory valve is attached to the inner ring-shaped valve seat and is able to seal against the outer ring-shaped valve seat. For discussion purposes the outer donut shaped flexible diaphragm portion or second expiratory valve opens at about 16 cmH₂O and closes at about 12 cmH₂O. Accordingly, in the event of a rapid increase in expiratory pressure and/or flow, the larger valve or valve portion would open to further increase the size of the opening for the expiration of air. Once the pressure within the passageway drops to around 12 cmH₂O, the larger valve or valve portion closes and leaves the smaller valve or valve portion open, to thereby maintain a positive end pressure within the apparatus.

The apparatus further includes an oxygen input port positioned upstream of the primary inspiratory valve or combination/two-stage inspiratory valve. In this way the collapsible reservoir bag is refilled from the source of oxygen or oxygen rich air when the primary inspiratory valve is closed.

The first inspiratory valve may open during expiration if the pressure within the collapsible reservoir bag reaches a predetermined level. This will mean that some of the oxygen rich air will be allowed to escape the apparatus to inhibit over pressurisation and rupturing of the collapsible reservoir bag. The first inspiratory valve will therefore act as a pressure release valve for the collapsible reservoir bag during expiration. The first inspiratory valve may open when the pressure within the collapsible reservoir bag reaches 16 centimetres of water (cmH₂O).

Therefore during expiration there may be a flow of air/gas out of the patient's airway into the passageway/s and through the expiratory valve, and out of the collapsible reservoir bag into the passageway/s and through the expiratory valve. Accordingly, under some circumstances the primary inspiratory valve would be open during expiration, although the direction of the flow of air/gas would still be exiting the apparatus through the expiratory valve.

Preferably the collapsible reservoir bag has a volume of between 100-600 millilitres (mL) and more preferably has a 500 millilitre (mL) volume. The collapsible reservoir bag may be secured over the inlet and attached to the body by use of an adhesive, or the bag may be heat welded to the body to inhibit it from becoming dislodged during use.

The collapsible reservoir bag may provide both visual and auditory evidence of respiration. In one form the collapsible reservoir bag is constructed from a material that emits a noise when it is being filled or emptied. The bag may be constructed from a polyethylene material and may be between 10-20 microns in thickness. In another form the passageway or passageways may include a noise emitting device that is activated when air flows thereover.

The coupling may be a female socket, having an opening extending therethrough, the socket may be configured to fit over the end of the ETT or tube of the LMA, and has an internal diameter of 15mm. The coupling may be tapered such that it frictionally engages the end of the ETT or tube of the LMA, or face mask or nasal airway respiratory support device, or the apparatus may be of unitary construction with the face mask or nasal airway respiratory support device. In another form the coupling is configured to engage a filter member positioned intermediate of the respiratory device and said apparatus.

The filter member may comprise a Heat Moisture Exchanger with Filter (HMEF) that uses the patient's own moisture and humidity from expiration to humidify anaesthetic gases during inspiration. Although it should be appreciated that other filters could also be used to inhibit saliva, condensation, or blood fouling the inspiratory or expiratory valves during use.

In one form the filter member is attached to the end of the ETT or tube of the LMA and the apparatus is attached to the filter member, wherein the filter member is intermediate of the apparatus and the ETT or LMA. Alternatively, the apparatus may include an integral filter member.

The coupling may be integral with the body portion and be of unitary construction, or the coupling may be connected to the body portion.

Preferably positive end expiratory pressure (PEEP) is used to maintain the pressure in the airway of the patient above atmospheric/ambient pressure. PEEP is produced in the patient by a non-complete or restricted exhalation, wherein the resistance to expiration is used to maintain the vocal cords open with a LMA *in situ.* PEEP also minimises airway and alveolar collapse distal to the vocal cords and increases compliance by increasing functional residual capacity (FRC). This improves oxygenation and reduces the degree of pulmonary shunt, where ventilation is mismatched to the lung perfusion, with either an ETT or LMA *in situ.*

Other ways of producing increased pressure in the lungs of the patent may be used such as, but not limited to, extrinsic PEEP that is applied by a ventilator, pressure support where pressure is applied or increased during an inspiration cycle, or continuous positive airway pressure (CPAP) where a pressure is applied at all stages of respiration to maintain an open airway.

In another aspect of the disclosure there is proposed a method of providing positive pressure to a patient's airway during recovery after anaesthesia or otherwise requiring respiratory support, including the steps of:
providing a disposable oxygenating apparatus including a body portion and a collapsible reservoir bag, the body portion having a passageway or passageways connecting an outlet, a coupling, a primary inlet and a secondary inlet, said collapsible reservoir bag attached over the primary inlet;
attaching said coupling at an outwardly extending end of an endotracheal tube or tube of a laryngeal mask airway, positioned within the airway of said patient, or to a face mask or a tube attached to said face mask, or a nasal airway respiratory support device, whereby said passageway or passageways are in fluid communication with the endotracheal tube, laryngeal mask airway, face mask or nasal airway respiratory support device,
filling the collapsible reservoir bag, from a source of oxygen or oxygen rich air, wherein upon inspiration by said patient a primary inspiratory valve, located in, or adjacent, the primary inlet, opens to permit the oxygen or oxygen rich air to be drawn in through the passageway or passageways from said collapsible reservoir bag and into the patient's airway, wherein if the collapsible reservoir bag is substantially emptied, a secondary inspiratory valve, located in, or adjacent the secondary inlet, opens to permit ambient air to be drawn in through said passageway or passageways from an exterior of said apparatus; and
refilling the collapsible reservoir bag during expiration by the patient, wherein the primary and secondary inspiratory valves are dosed for at least a period of time during expiration and an expiratory valve positioned in or adjacent the outlet opens to permit movement of an expired air therethrough, the expiratory valve being configured to close at a selected pressure to maintain positive pressure in the patient's airway relative to an ambient atmospheric pressure.

The method may include the additional step of adjusting the expiratory valve by way of an adjustment member or the expiratory valve being an adjustable expiratory valve to maintain a desired pressure within the patient's airway.

### BRIEF DESCRIPTION OF THE DRAWINGS

The accompanying drawings, which are incorporated in and constitute a part of this specification, illustrate an implementation of the invention and, together with the description and claims, serve to explain the advantages and principles of the invention. In the drawings,
- Figure 1: is a perspective view of one embodiment of the oxygenating apparatus of the present invention;

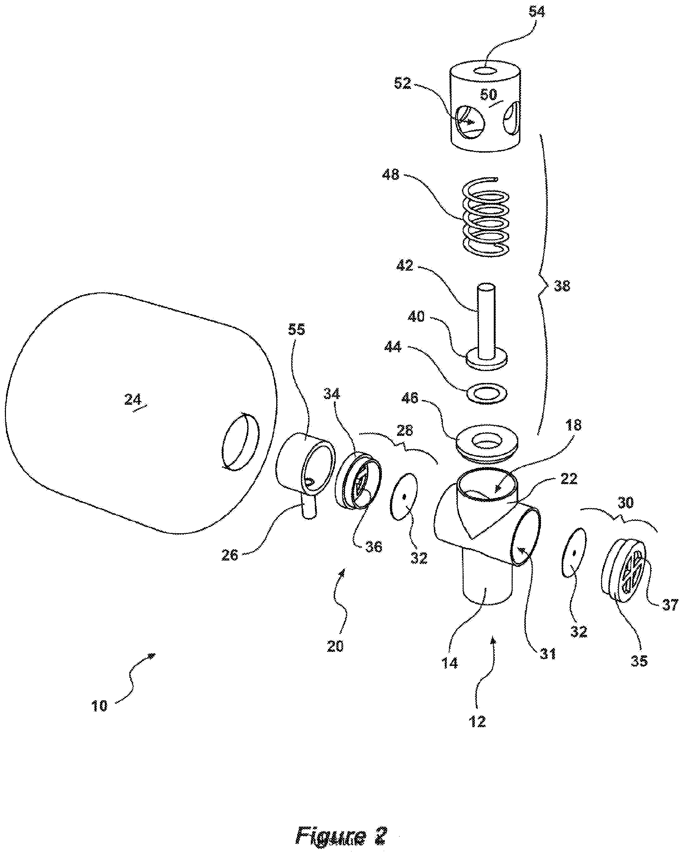

- Figure 2: is an exploded view of the oxygenating apparatus of figure 1;

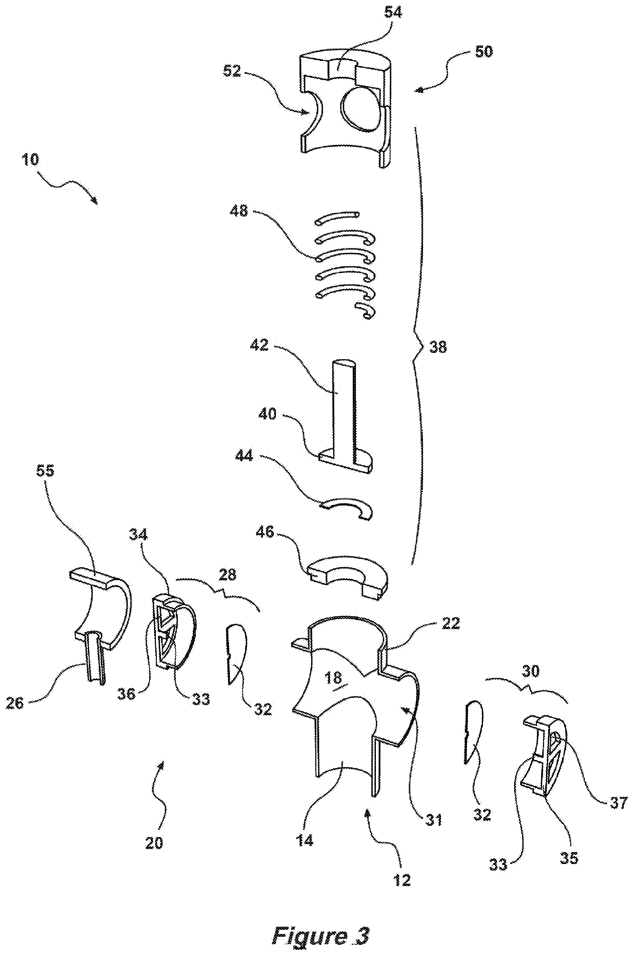

- Figure 3: is a cross-sectional view of figure 2 illustrating the body and valves;
- Figure 4: is a schematic view of the oxygenating apparatus of figure 1, illustrating the opening of the expiratory valve during expiration by a patient;
- Figure 5: is a schematic view of the oxygenating apparatus of figure 1, illustrating the closing of the expiratory valve during the later stage of expiration;
- Figure 6: is a schematic view of the oxygenating apparatus of figure 1, illustrating the opening of the primary inspiratory valve during inhalation by the patient;
- Figure 7: is a schematic view of the oxygenating apparatus of figure 1, illustrating the opening of the secondary inspiratory valve;
- Figure 8a: is a possible embodiment of the combination valve in fluid communication with both the ambient air and passageway, illustrating exhaled air being expelled from the apparatus;
- Figure 8b: is the combination valve of figure 8a illustrating entry of ambient air in through the passageway;
- Figure 9: is a perspective view of a possible embodiment of a two-stage inspiratory valve;
- Figure 10: is a cross-sectional view of the two-stage inspiratory valve of figure 9;
- Figure 11: is an exploded view of the two-stage inspiratory valve of figure 9;
- Figure 12: is a partial cross-sectional view of valve of figure 11;
- Figure 13: is a perspective view of the oxygenating apparatus illustrating two-stage expiratory valve;
- Figure 14: is a cut away view of the expiratory valve of figure 13;
- Figure 15a: is a schematic view of the expiratory valve of figure 13 in a closed arrangement;
- Figure 15b: is a schematic view of the expiratory valve of figure 15a in a partially open arrangement;
- Figure 15c: is a schematic view of the expiratory valve of figure 15a in a completely open arrangement;
- Figure 16: is a schematic view of the apparatus attached to a LMA; and
- Figure 17: is a schematic view of the apparatus attached to a face mask of a non-invasive positive pressure device.

### DETAILED DESCRIPTION OF THE ILLUSTRATED AND EXEMPLIFIED EMBODIMENTS

Similar reference characters indicate corresponding parts throughout the drawings. Dimensions of certain parts shown in the drawings may have been modified and/or exaggerated for the purposes of clarity or illustration. Although the detailed description of the invention is directly primarily to the use of ETT and LMA on anaesthetised patients, the reader should appreciate that this is in no way intended to limit the scope of the invention, and the same or similar advantages are envisaged when the apparatus is used on non-invasive ventilation support face masks and nasal airway respiratory support devices.

Referring to the drawings for a more detailed description, there is illustrated a disposable oxygenating apparatus 10, demonstrating by way of examples, arrangements in which the principles of the present invention may be employed. The apparatus 10 includes a body portion 12 having a coupling 14 for attachment to an end 16 of an endotracheal tube, a tube of a laryngeal mask airway, face mask or nasal airway respiratory support device. A passageway 18 extends through the body portion 12 and connects the coupling 14 with primary and secondary inlets 20, 31, and an outlet 22.

A collapsible reservoir bag 24 is attached over the primary inlet 20 and is in fluid communication with a source of oxygen or oxygen rich air 25, by way of input port 26 that is located upstream of a primary inspiratory valve 28. The collapsible reservoir bag 24 is refilled from the source of oxygen or oxygen rich air 25 when the primary inspiratory valve 28 is closed. It should be appreciated that the inflow of oxygen is generally constant, but can be varied or the volume adjusted by the medical personnel.

The apparatus 10 further includes a secondary inspiratory valve 30 in, or adjacent, the secondary inlet 31 in a side of passageway 18 for controlling entry of ambient air from the exterior of the apparatus, in through the passageway 18 during inspiration when the collapsible reservoir bag 24 has been substantially emptied.

The secondary inspiratory valve 30 acts as a safety valve to ensure that the patient is provided with inhalable air at all times. This configuration inhibits the patient from performing a 'Valsalva' manoeuver, being a forced inspiration against a closed glottis or obstruction, which can be dangerous to the patient.

In the present embodiment both the primary and secondary inspiratory valves 28, 30 are diaphragm valves, each comprising a respective flexible rubber diaphragm 32 attached to a central protrusion 33 of a respective valve mount 34, 35, having apertures 36 or 37 extending therethrough.

An expiratory valve 38 is located within, or adjacent, the outlet 22 and is configured to control the passage of an expired gas out through the apparatus 10 during expiration. In the present embodiment the expiratory valve 38, is an in-line check valve assembly comprising a disc 40 attached to a shaft 42. The disc 40 having a resiliently flexible sealing member 44 attached to a front face, for abutment with an annular valve seat 46. A helical spring 48 is positioned around the shaft 42 for biasing the flexible sealing member 44 against the valve seat 46. The components of the expiratory valve 38 are held within valve housing 50 that includes outlet apertures 52 for passage of the expired air and an orifice 54 that slidably locates shaft 42.

The expiratory valve 38 is biased closed by the helical spring 48 to maintain a pressure within the airway of the patient of between 8-12 centimetres of water (cmH₂O). This positive end expiratory pressure assists in keeping the vocal cords open during intubation with a LMA and minimises airway and alveolar collapse when the ETT or LMA is positioned within the airway of the recovering patient.

The size and configuration of the outlet apertures 52 permit the expired air to rapidly dissipate, even in the event that the patient coughs.

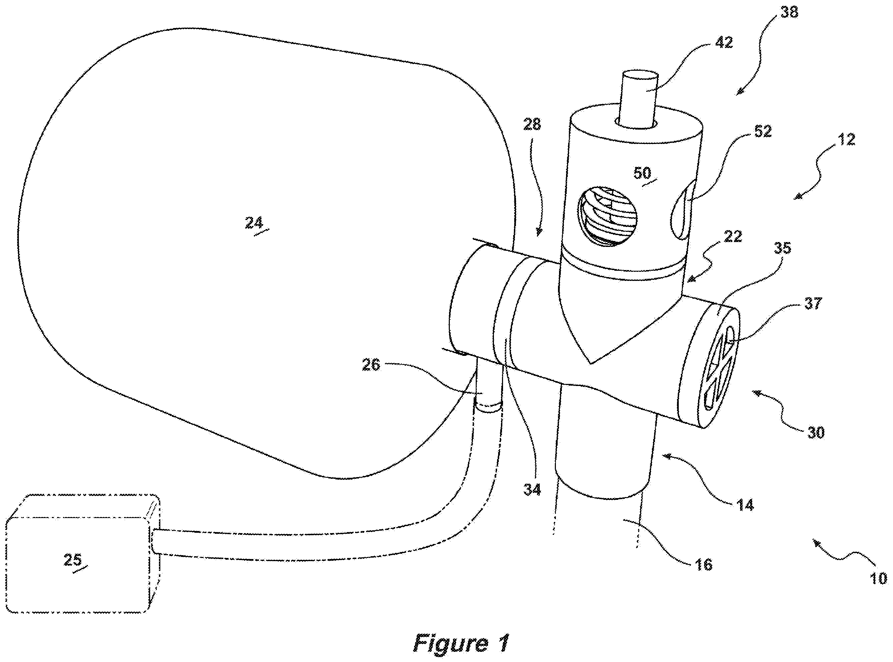

The apparatus 10 includes collar 55 that incorporates the input port 26 and to which the opening of the collapsible reservoir bag 24 can be heat welded or glued as illustrated in figure 1.

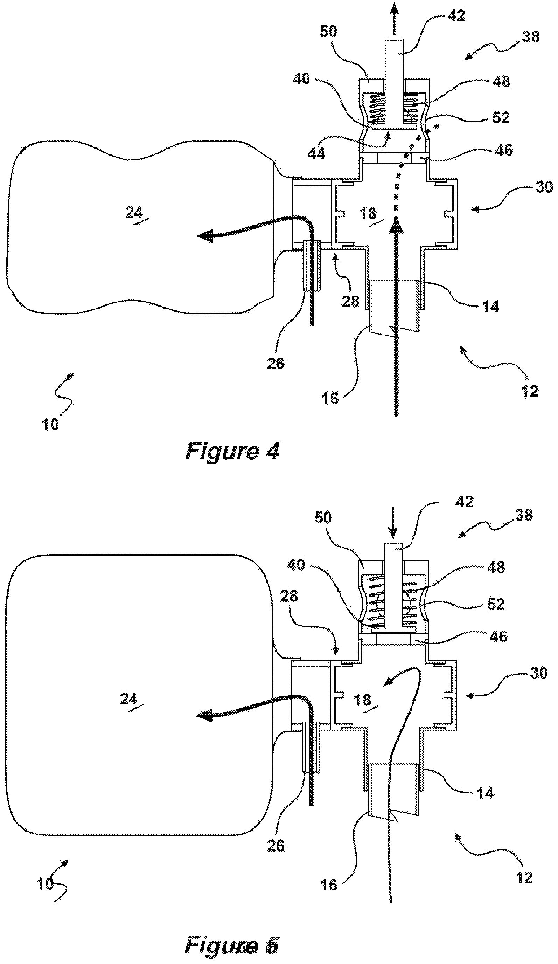

Turning to figures 4 to 7 there is illustrated the different stages of airflow through the apparatus 10 during use. During expiration, pressure within the passageway 18 increases which causes the expiratory valve 38 to open against the influence of the helical spring 48, as shown in figure 4. The disc 40 moves off the annular valve seat 46 in the direction indicated by the arrow, thereby allowing expired air to move through the passageway 18 and out through outlet apertures 52 into the surrounding environment.

If the patient breathes out rapidly or with a large volume, such as in the event the patient coughs, the expiratory valve 38 opens fully to allow the air to flow through the sidewardly open outlet apertures 52. Therefore the greater the pressure generated within the passageway 18 of the apparatus by the expiration of air, the further open the expiratory valve 38 is pushed and the faster the pressure drops back to the desired 8-12 centimetres of water (cmH₂O) within the passageway 18 or airway of the patient.

It is important that the apparatus 10 can accommodate rapid rises in airflow to inhibit either injury to the patient, or damage/dislodgement of the oxygenation apparatus, ETT or LMA.

As illustrated in figure 5, during the final stages of expiration, as the expired air flow from the patient's airway decreases, the spring 48 forces the disc 40 to close against the annular seat 46, as indicated by the arrow, to thereby close the expiratory valve 38. This thereby maintains a positive end pressure within the passageway 18 of between 8-12 centimetres of water (cmH₂O).

The collapsible reservoir bag 24 is in constant fluid communication with the source of oxygen or oxygen rich air 25 and is filled by way of input port 26 as indicated by the arrow when the primary inspiratory valve 28 is closed. As indicated in figure 5 during the expiration cycle, and also between breaths, the collapsible reservoir bag 24 continues to be filled with oxygen. The reader should appreciate that the flow of oxygen rich air in through the input port 26 is preferably constant, however a medical practitioner may vary the flow rate by way of conventional means. In the present embodiment 100% oxygen is used and the bag has a capacity of 500mL. The reader should however appreciate that the oxygen rich air from the source does not need to be 100% oxygen.

As the reader will now appreciate, during inspiration, there are essentially two sources from which the patient receives breathable air or gas. Preferentially, the patient receives oxygen or oxygen rich air through the primary inspiratory valve 28 from the source of oxygen or oxygen rich air 25 at least partially via the collapsible reservoir bag 24. Then, if the bag 24 is substantially emptied, ambient air is drawn in through the secondary inspiratory valve 30 from the surrounding environment.

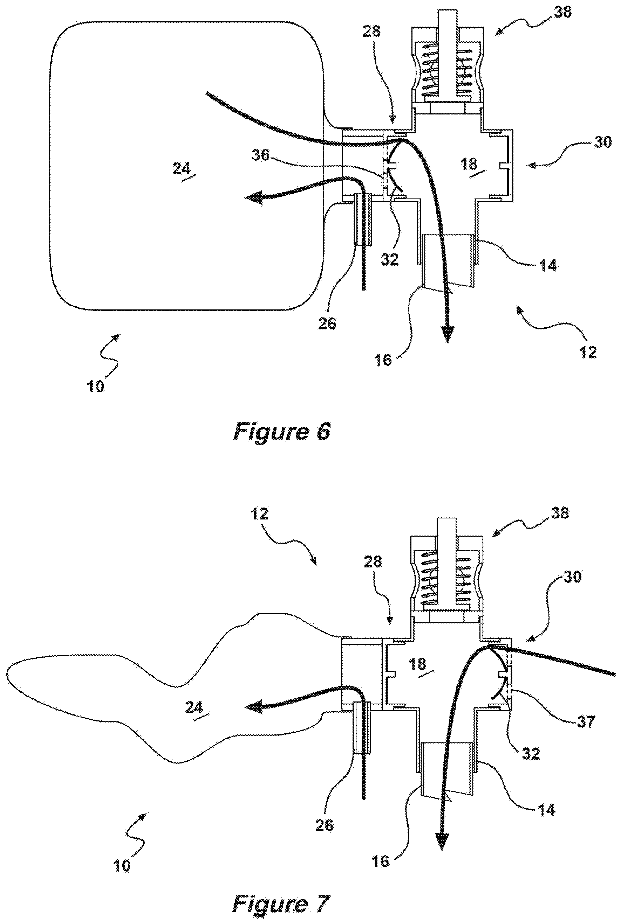

When the patient begins to inhale, as illustrated in figure 6, the diaphragm 32 of the primary inspiratory valve 28 is drawn away from mount 34 by the reduction in pressure within passageway 18. This opens apertures 36 and permits the oxygen or oxygen rich air within the collapsible reservoir bag 24 to be drawn in through the apparatus 10 and into the patient's airway.

Typically a sedated or recovering patient will have a tidal volume equal to, or less than 500mL. Accordingly, a 500ml bag will be sufficient so that the patient will inspire close to 100% from the bag, without entraining any ambient air through the primary inspiratory valve 30. However for patients who have large lung capacities or those in pain, the secondary inspiratory valve 30 is provided so that the remaining percentage of required air for inspiration can be drawn in from the surrounding environment, exterior of the apparatus 10, while still permitting the apparatus to maintain pressure to the airway of the patient when the secondary inspiratory valve 30 closes.

As illustrated in figure 7, when the collapsible reservoir bag 24 has been substantially emptied the diaphragm 32 of the secondary inspiratory valve 30 is drawn away from mount 35 to thereby open corresponding apertures 37, which allows ambient air to the drawn into the passageway 18 from the surrounding environment, as indicated by the solid arrow.

Although the fraction of oxygen (FiO₂) is decreased by the opening of the secondary inspiratory valve 30, it means that the collapsible reservoir bag does not need to be of too large volume to cope will all patients or circumstances.

As the reader will now appreciate, when the pressure on the upstream side of the primary inspiratory valve 28 is greater than the pressure on the downstream side of the primary inspiratory valve 28, which occurs during inspiration by the patient, the valve will open allowing the oxygen or oxygen rich air in the collapsible reservoir bag 24 to flow in through the passageway 18 and in turn into the airway of the patient. During expiration by the patient the pressure on the downstream side of valves 28, 30 will increase thereby closing the respective flexible rubber diaphragm 32 over the aperture/s 36, 37 of valve mounts 34, 35.

The use of a diaphragm check valve also means that if the volumetric flow rate of oxygen or oxygen rich air from the source 25 is set too high, the flexible diaphragm 32 of the primary inspiratory valve 28 will open to permit excess gas to escape during the expiration cycle. This will inhibit the bag 24 from tearing due to over-pressurisation.

The coupling 14 of the present embodiment is a female socket that is configured to fit over the end 16 of the ETT/LMA and has an internal diameter of 15mm. The coupling 14 may be tapered so that it can frictionally engage the end 16 of the ETT/LMA.

As illustrated in the figures the primary and secondary inspiratory valves 28 and 30 are recessed into the side of the passageway 18 to inhibit saliva, condensation, or blood fouling the valves during use.

The primary and secondary inspiratory valves 28, 30 may be the same size provided the primary valve 28 opens first and allows the collapsible reservoir bag 24 to be substantially emptied before the secondary valve 30 opens.

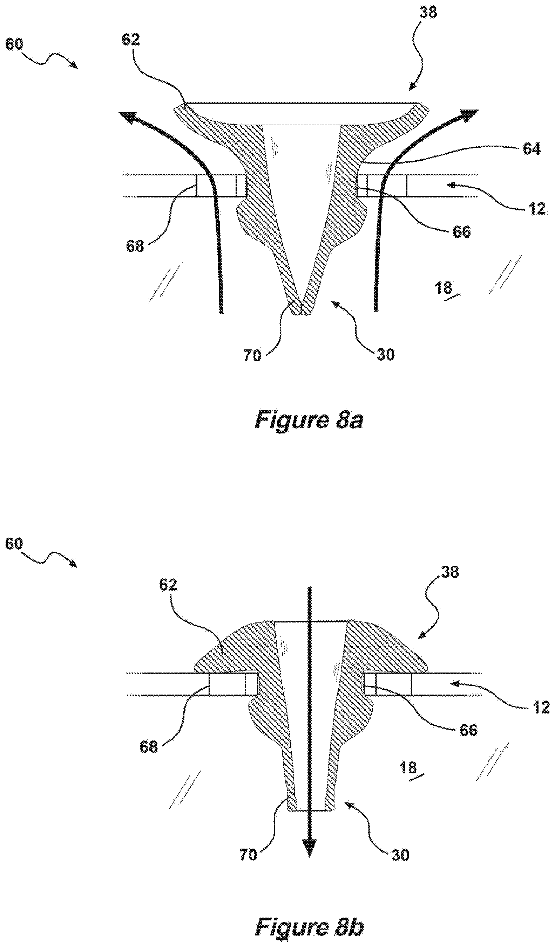

The expiratory valve 38 and the secondary inspiratory valve 30 may form parts of a combination valve 60. In one possible embodiment as illustrated in figures 8a and 8b, the expiratory valve 38 includes a flexible skirt 62 and a central portion 64 secured through aperture 66 in the side of body portion 12 and at rest seals openings 68. The secondary inspiratory valve 30 includes a soft tube 70 that is closed by back-pressure within passageway 18 to cut off a flow of air

In this way during expiration the pressure within the passageway 18 increases to a point that causes the soft tube 70 to close and the flexible skirt 62 to open to thereby permit the expired air to be expelled through the openings 68, as illustrated in figure 8a. During inspiration the pressure within the passageway 18 decreases which causes the flexible skirt 62 to close over openings 68. In the event the collapsible reservoir bag is substantially emptied during inspiration the pressure drops to a point that the soft tube 70 will open to permit the entry of ambient air therethrough as illustrated in figure 8b.

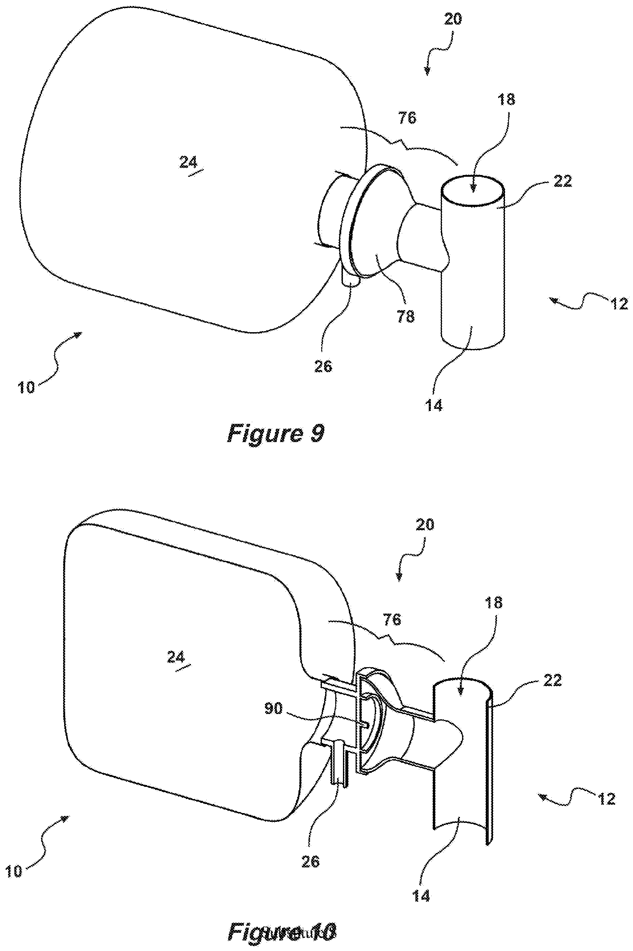

In another possible embodiment, as illustrated in figures 9 to 12, the primary and secondary inspiratory valves are replaced by a two-stage inspiratory valve 76, which is located within casing 78. The two-stage inspiratory valve 76 includes a disc shaped flexible diaphragm 80, and a ring-shaped flexible diaphragm 82, wherein the disc shaped flexible diaphragm 80 is located centrally of the ring-shaped flexible diaphragm 82.

The flexible diaphragms 80 and 82 are attached to frame 84, the frame including central apertures 86 extending therethrough, in fluid communication with the collapsible bag 24, and peripheral apertures 88 in fluid communication with the ambient air. The disc shaped flexible diaphragm 80 is configured to reversibly seal central apertures 86 and the ring-shaped flexible diaphragm 82 is configured to reversibly seal peripheral apertures 88.

The two-stage inspiratory valve 76 is configured so that the disc shaped flexible diaphragm 80 opens first, whereby air is preferentially drawn from within the collapsible bag 24, during inspiration. In the event that the collapsible bag 24 is substantially emptied the ring-shaped flexible diaphragm 82 moves to open peripheral apertures 88 to allow ambient air to be drawn in from the exterior of the apparatus 10.

In this way the two-stage inspiratory valve 76 incorporates the functionality of the first and secondary inspiratory valve in a single unit. The disc shaped flexible diaphragm 80 may be attached to a central protrusion 90 and the ring-shaped flexible diaphragm 82 may be attached to the frame 84 adjacent the disc shaped flexible diaphragm 80, wherein the outer edge of ring-shaped flexible diaphragm 82 is permitted to flex.

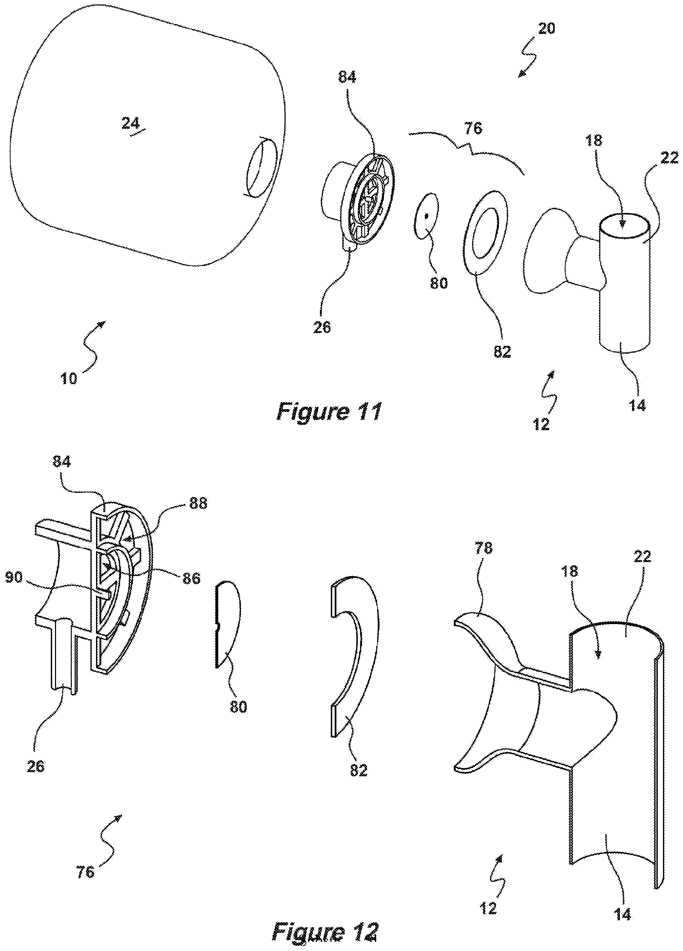

As illustrated in figure 12, the flexible diaphragms 80 and 82 are of different thicknesses, wherein the disc shaped flexible diaphragm 80 opens first under negative pressure within passageway 18, and the ring-shaped flexible diaphragm 82 opens secondly, when the pressure drops further within passageway 18, as would occur when the collapsible bag 24 is substantially emptied. It should however be appreciated that different thicknesses are not the only way to cause the preferential opening of one inspiratory valve, and other ways of preferential opening could be used, including but not limited to, material and size of the valves.

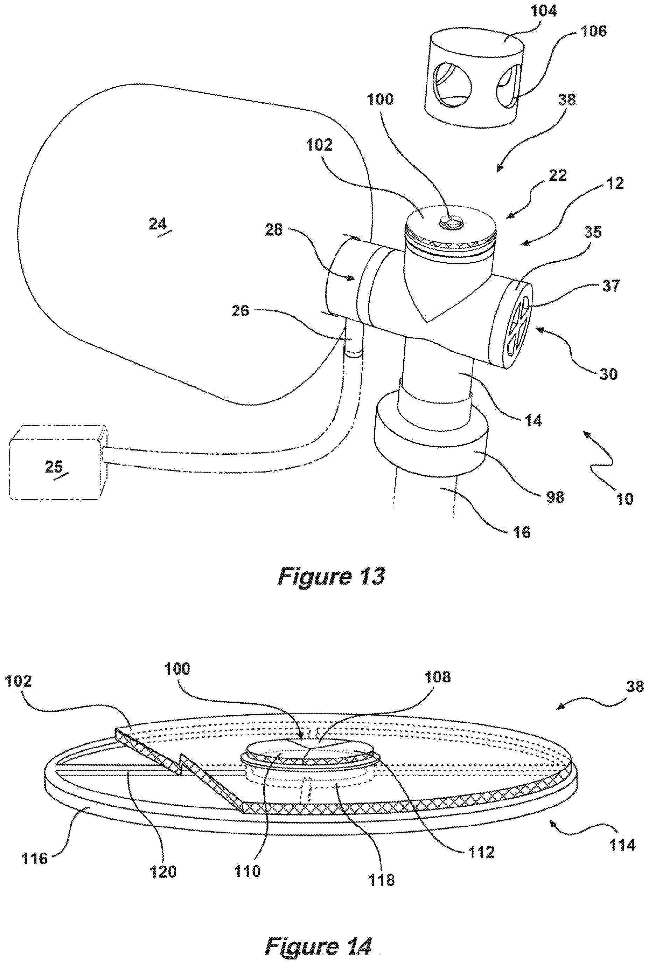

Figure 13 illustrates one embodiment of a two-stage expiratory valve comprising a central flexible portion 100, a larger outer donut shaped flexible diaphragm portion 102, which are protected by cover 104 having sidewardly open apertures 106. The central flexible portion 100 includes three cooperating leaflets 108, 110, 112.

Figure 13 also illustrates a filter member 98, in the present embodiment being a heat moisture exchanger with filter (HMEF). The filter member 98 is positioned intermediate of the end 16 of the endotracheal tube or laryngeal mask airway and the apparatus 10. The reader should however appreciate that the apparatus 10 may include an integral filter or filters to inhibit fouling the valves by saliva, condensation, or blood.

As further illustrated in Figure 14, the central flexible portion 100, and the larger outer donut shaped flexible diaphragm portion 102 are attached to and supported on valve fame 114, which includes an outer ring-shaped valve seat 116, connected to an inner ring-shaped valve seat 118 by radially extending arms 120. The outer ring-shaped valve seat 116 may be around 5cm in diameter and the inner ring-shaped valve seat 118 may be around 2cm in diameter.

In the present embodiment, the central flexible portion 100 is attached to the top of inner ring-shaped valve seat 118, and the outer donut shaped flexible diaphragm portion 102 engages with, or is attached to a side of the inner ring-shaped valve seat 118, wherein inner parts of the cooperating leaflets 108, 110, 112 are able to flex and outer parts of donut shaped flexible diaphragm portion 102 are able to flex.

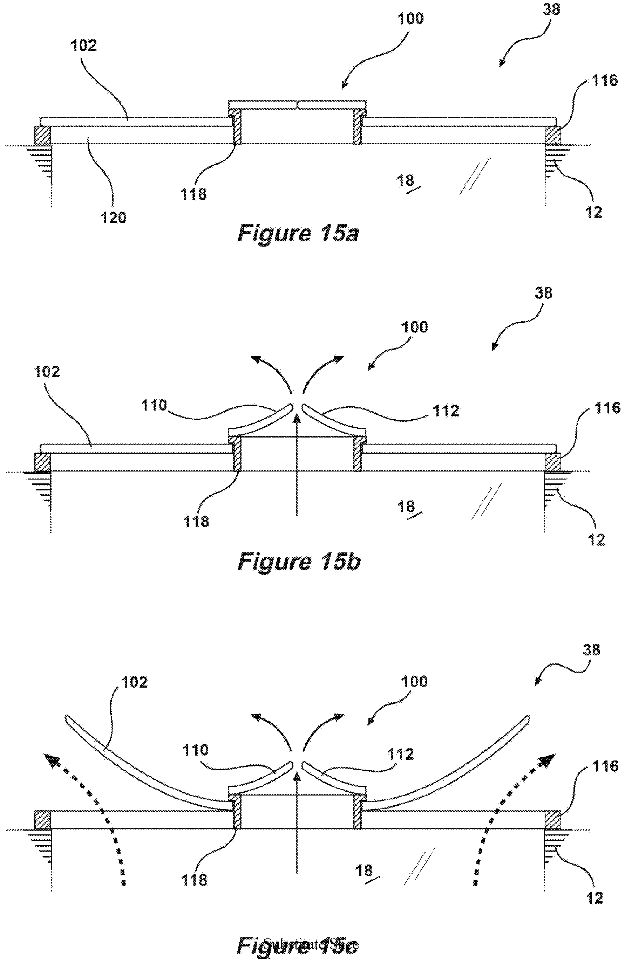

As shown in Figures 15a to 15c the central flexible portion 100 and larger outer donut shaped flexible diaphragm portion 102 open progressively as the pressure within passageway 18 increases during expiration.

Figure 15a illustrates the arrangement of the expiratory valve 38 when the patient is at rest, i.e. between breaths, or when they are inhaling. When the patient is exhaling under normal situations, the cooperating leaflets 108, 110, 112, open when the pressure within passageway 18 reaches around 12 cmH₂O to permit passage of the expired air, as indicated by the solid arrows in Figure 5b.

In the event that the patient coughs, the arrangement of the expiratory valve 38 changes, as illustrated in Figure 15c, such that the outer donut shaped flexible diaphragm portion 102 opens, at around 16 cmH₂O, to thereby rapidly dissipate the expired air, as indicated by the broken arrows, to thereby inhibit injury to the patient or dislodgement of the apparatus, ETT or LMA. The cooperating leaflets 108, 110, 112, also remain open and expired air passes therebetween, as indicated by the solid arrows. The reader should however appreciate that other types of combination valves or two-stage valves could be used without departing from the scope of the invention. Furthermore, a plurality of outlet/expiratory or inlet/inspiratory valves may be used.

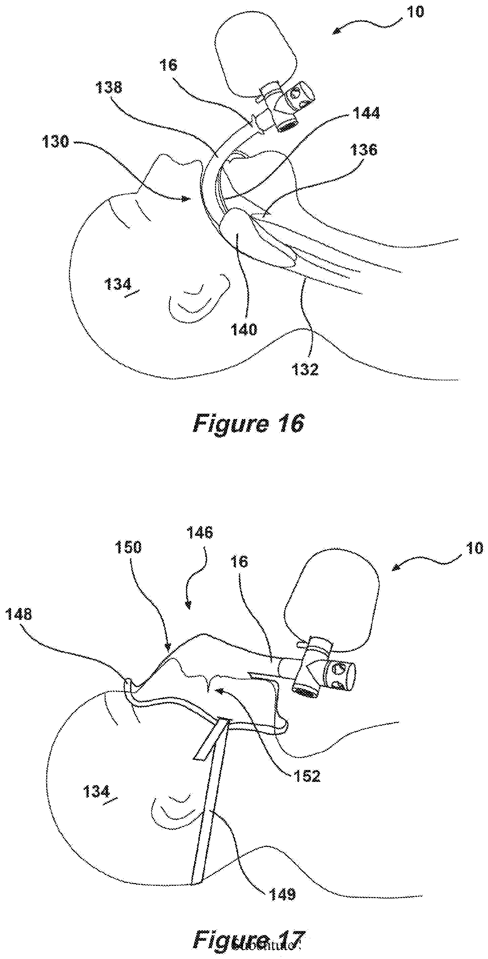

Figure 16 illustrates use of the apparatus 10 on a LMA 130 that is positioned within the airway 132 of a patient 134 adjacent the epiglottis 136. The LMA 130 includes a pipe 138 having end 16, an inflatable cuff 140, and inflation line 144.

The apparatus can also be attached to a face mask 146, such as, but not limited to, that of a non-invasive positive pressure device such as those used on patients suffering from heart failure or chronic obstructive pulmonary disease, as illustrated in Figure 17. The face mask 146 includes an inflated or pneumatic skirt 148 and strap 149, and covers the nose 150 and mouth 152 of the patient during use. The reader should appreciate that the apparatus and face mask may be of unitary construction, or the apparatus and nasal airway respiratory support device may be of unitary construction.

The apparatus 10 is connected directly to the tight fitting face mask 146 in Figure 17, however it should be appreciated that the apparatus 10 could be connected to a flexible tube (not shown) connected to the face mask 146 worn by the patient. Accordingly the spontaneously breathing patient wearing the face mask 146 would get increased FiO₂ and some PEEP.

The use of the apparatus 10 has significant advantages over the existing Continuous Positive Airway Pressure (CPAP) and Bi-Level Positive Air Pressure (BiPAP) systems that can be expensive and require patient tolerance, which is sometimes difficult to achieve.

It should also be appreciated that the phrase 'fluid communication', used throughout the specification relates to the flow of air through the apparatus between the airway of the patient and the collapsible bag and exterior environment.

The skilled addressee will now appreciate the advantages of the illustrated invention over the prior art. In one form the invention provides a disposable oxygenation apparatus that can be used to apply an oxygen enriched, positive pressure to a patient's airway when an ETT or LMA is *in situ,* to thereby decrease the incidence of desaturation, and with a LMA in situ, assist in maintaining the vocal cords of the patient open during recovery after anaesthesia. The apparatus also inhibits injury to a patient or dislodgement of the oxygenation apparatus, ETT or LMA, when a patient coughs.

## Claims

1. A disposable oxygenating apparatus (10) for use on a patient recovering from anaesthesia or otherwise requiring respiratory support, where a respiratory device is in situ or is to be used, including,
a body portion (12) having a coupling (14) for attachment to said respiratory device an end (16) of an endotracheal tube or a tube of said laryngeal mask airway, which extends outwardly from an airway of said patient,
a primary inlet (20),
a secondary inlet (31),
an outlet (22),
a passageway (18) or passageways for fluid communication with the respiratory device, the passageway (18) or passageways extending through said body portion (12) between said coupling (14) and both the primary inlet (20) and the secondary inlet (31), and extending through said body portion (12) between said coupling (14) and the outlet (22), for fluid communication therebetween,
a collapsible reservoir bag (24) attached over said primary inlet (20), said collapsible reservoir bag in fluid communication with a source of oxygen or oxygen rich air (25), an expiratory valve (38) or valves located in or adjacent said outlet (22) for controlling the passage of an expired air from said patient during expiration, wherein a positive pressure is applied to the airway of said patient during expiration and rest,
a primary inspiratory valve (28) located in or adjacent said primary inlet (20), for controlling said oxygen or oxygen rich air flowing in through said passageway (18) or passageways from said collapsible reservoir bag (24), for at least a period of time during inspiration by said patient, and
a secondary inspiratory valve (30) located in or adjacent said secondary inlet (31) in fluid communication with said passageway (18) or passageways for controlling entry of an ambient air in through the passageway or passageways from an exterior of said apparatus during inspiration when said collapsible reservoir bag (24) has been substantially emptied.

2. The apparatus (10) according to claim 1, wherein the respiratory device is an endotracheal tube or laryngeal mask airway, wherein the apparatus is attached at an end of an endotracheal tube or a tube of said laryngeal mask airway, which extends outwardly from an airway of said patient, or the respiratory device includes a face mask that it attached over a mouth and a nose of said patient, wherein the apparatus is attached to said face mask or a tube connected to said face mask, or the respiratory device comprises a nasal airway respiratory support device.

3. The apparatus (10) according to claim 1 or 2, wherein the expiratory valve (38) located in or adjacent said outlet (22), is an in-line check valve, or the expiratory valve comprises a two-stage expiratory valve comprising a central flexible portion (100), and a larger outer donut shaped flexible diaphragm portion (102), the central flexible portion (100) including a plurality of cooperating leaflets (108, 110, 112), whereby the central flexible portion *i.e.* first expiratory valve opens first, and air is preferentially expelled therethrough, from within the passageway (18) or passageways during expiration, and if said patient coughs the outer donut shaped flexible diaphragm portion (102) *i.e.* second expiratory valve opens to thereby rapidly dissipate the expired air, *i.e*. the expiratory valve (38) comprises cooperating first and second expiratory valves.

4. The apparatus (10) according to claim 3, wherein the plurality of cooperating leaflets (108, 110, 112) or first expiratory valve opens at between 10 centimetres of water (cmH₂O) and 14 cmH₂O: or at 12 cmH₂O, and closes at between 6 cmH₂O and 10 cmH₂O, or 8 cmH₂O, and the outer donut shaped flexible diaphragm portion (102) or second expiratory valve opens at between 14 cmH₂O and 18 cmH₂O, or 16 cmH₂O, and close at between 10 cmH₂O and 14 cmH₂O, or at 12 cmH₂O, to thereby maintain a positive end pressure within the apparatus while rapidly dissipating the expired air when a volumetric flow rate of expired air increases.

5. The apparatus (10) according to claim 2 or claims 3 or 4 as appended to claim 2, wherein the expiratory valve (38) maintains a positive end pressure and opens at low pressure to inhibit over pressurisation of the patient's airway, to thereby maintain a pressure within the airway of the patient of between 4-14 centimetres of water (cmH₂O), or 8 cmH₂O.

6. The apparatus (10) according to any preceding claim, wherein the primary and secondary inlets (20, 31) are spaced apart from each other at different locations along a single passageway (18), or are located along different passageways, or are located at the same location along said single passageway (18).

7. The apparatus (10) according to any preceding claim, wherein the primary inspiratory valve is a diaphragm check valve including a flexible rubber diaphragm positioned over at least one aperture in the primary inlet and the secondary inspiratory valve is a diaphragm check valve including a flexible rubber diaphragm positioned over at least one aperture in the secondary inlet.

8. The apparatus (10) according to claim 7, wherein the primary inspiratory valve (28) has a lower opening pressure compared to the secondary inspiratory valve (30), such that the primary inspiratory valve opens before the secondary inspiratory valve, whereby oxygen or oxygen rich air is preferentially inhaled from within the collapsible reservoir bag (24), and when said collapsible reservoir bag is substantially emptied the secondary inspiratory valve (30) opens to provide the patient with a source of breathable air.

9. The apparatus (10) according to claim 8, wherein said primary and secondary inspiratory valves (28, 30) are incorporated in a combination valve or a two-stage inspiratory valve (76), said combination or two-stage inspiratory valve being located within a casing (78) having a frame (84) including central apertures (86) extending therethrough, in fluid communication with the collapsible reservoir bag (24), and peripheral apertures (88) in the frame in fluid communication with the ambient air, whereby the primary inspiratory valves (28) includes a disc shaped flexible diaphragm (80) being attached to said frame (84) and configured to reversibly seal the central apertures (86), and the secondary inspiratory valve (30) includes a ring-shaped flexible diaphragm (82) being attached to said frame (84) and configured to reversibly seal the peripheral apertures (88).

10. The apparatus (10) according to claim 2 or any one of claims 3 to 9 as appended to claim 2, wherein the expiratory valve (38), primary inspiratory valve (28) and secondary inspiratory valve (30) each comprises a check valve or one-way valve, being selected from a group containing diaphragm check valves, ball check valves, duckbill valves, tilting disc check valves, lift-check valves and in-line check valves.

11. The apparatus (10) according to any preceding claim, wherein the collapsible reservoir bag (24) has a volume of between 100-600 millilitres (mL), or 500 mL, and is secured to the body over the primary inlet (20) by use of an adhesive, or the collapsible reservoir bag is heat welded or otherwise affixed to the body.

12. The apparatus (10) according to claim 2 or any one of claims 3 to 11 as appended to claim 2, wherein the first inspiratory valve opens during expiration if a pressure within the collapsible reservoir bag (24) reaches a predetermined level, whereby at least some of the oxygen rich air from within the collapsible reservoir bag is permitted to flow out into said passageway or passageways and out through the expiratory valve, to thereby inhibit rupturing of the collapsible reservoir bag.

13. The apparatus (10) according to claim 11, wherein the collapsible reservoir bag (24) provides auditory evidence of respiration, wherein the collapsible reservoir bag emits a noise when it is being filled or emptied, by way of a pneumatic noise emitting device that is activated when airflows therethrough, or said noise is emitted due to the material from which the collapsible reservoir bag is constructed.

14. The apparatus (10) according to any preceding claim, wherein the coupling (14) being configured to engage a filter member (98) positioned intermediate of the respiratory device and said apparatus, the coupling (14) being integral with the body portion (12), or of unitary construction, or the coupling being connected to the body portion.

## Patentansprüche

1. Einweg-Sauerstoffanreicherungsvorrichtung (10) zur Verwendung bei einem Patienten, der sich davon erholt Anästhesie oder anderweitig eine Atmungsunterstützung erforderlich, wenn ein Atemweg Gerät ist in situ oder soll verwendet werden, einschließlich
einen Körperabschnitt (12) mit einer Kupplung (14) zum Anbringen an dem Atmungsorgan Vorrichtung ein Ende (16) eines Endotrachealtubus oder eines Tubus der Kehlkopfmaske Luftweg, der sich von einem Luftweg des Patienten nach außen erstreckt,
einen primären Einlass (20),
einen sekundären Einlass (31),
einen Auslass (22),
einen Durchgang (18) oder Durchgänge für die Fluidverbindung mit der Atemwege Vorrichtung, wobei sich der Durchgang (18) oder Durchgänge erstrecken durch den Körperabschnitt (12) zwischen der Kupplung (14) und sowohl dem primären Einlass (20) als auch dem sekundären Einlass (31) und sich durch den Körperabschnitt (12) zwischen der Kupplung (14) und dem Auslass (22) erstrecken für die fließende Kommunikation dazwischen,
einen zusammenlegbaren Reservoirbeutel (24), der über dem Primäreinlass (20) angebracht ist, wobei der faltbarer Reservoirbeutel in Fluidverbindung mit einer Sauerstoffquelle oder sauerstoffreiche Luft (25), ein Ausatmungsventil (38) oder Ventile, die in oder nebeneinander angeordnet sind den Auslass (22) zum Steuern des Durchlasses einer ausgeatmeten Luft von dem Patienten während des Ausatmens, wobei ein Überdruck auf den Luftweg des besagten ausgeübt wird Patient während des Ausatmens und der Ruhezeit,
ein primäres Inspirationsventil (28), das in oder neben dem Primäreinlass (20) angeordnet ist, zur Steuerung der durch Sauerstoff einströmenden sauerstoff- oder sauerstoffreichen Luft Durchgangswege (18) oder Durchgangswege von dem zusammenlegbaren Reservoirbeutel (24), mindestens eine Zeitspanne während der Inspiration durch den Patienten, und
ein sekundäres Inspirationsventil (30), das in oder neben dem sekundären Einlass angeordnet ist (31) in Fluidverbindung mit dem Durchgang (18) oder Durchgängen für Steuern des Eintritts einer Umgebungsluft durch den Durchgang oder die Durchgänge von der Außenseite der Vorrichtung während des Einatmens, wenn das zusammenfaltbare Der Reservoirbeutel (24) wurde im Wesentlichen entleert.

2. Vorrichtung (10) nach Anspruch 1, wobei das Beatmungsgerät ein ist Endotrachealtubus oder Larynxmasken-Luftweg, wobei die Vorrichtung angebracht ist an einem Ende eines Endotrachealtubus oder eines Tubus des Kehlkopfmaskenluftwegs, der sich von einem Atemweg des Patienten nach außen erstreckt, oder das Beatmungsgerät umfasst eine Gesichtsmaske, die über einem Mund angebracht ist, und eine Nase des Patienten, wobei die Vorrichtung an der Gesichtsmaske oder einem Schlauch befestigt ist mit der Gesichtsmaske verbunden ist, oder das Beatmungsgerät eine Nase umfasst Atemwegsunterstützungsvorrichtung für die Atemwege.

3. Vorrichtung (10) nach Anspruch 1 oder 2, wobei das Ausatmungsventil (38) in oder neben dem Auslass (22) angeordnet ist, ist ein Inline Rückschlagventil oder das Ausatmungsventil umfasst ein zweistufiges Ausatmungsventil, umfassend einen zentralen flexiblen Abschnitt (100) und eine größere äußere ringförmige Membran Abschnitt (102), wobei der zentrale flexible Abschnitt (100) eine Vielzahl von zusammenwirkenden Blättchen (108, 110, 112) umfasst, wodurch der zentrale flexible Abschnitt, d.h. das erste Ausatmungsventil, zuerst geöffnet wird und Luft vorzugsweise aus dem Innern hindurch ausgestoßen wird Durchgangswege (18) oder Durchgangswege während des Ausatmens, und wenn der Patient den äußeren ringförmigen Membranabschnitt (102) hustet, d.h. das zweite Ausatmungsventil öffnet, um dadurch die ausgeatmete Luft schnell abzuführen, d.h. das Ausatmungsventil (38) umfasst ein zusammenwirkendes erstes und zweites Ausatmungsventil Ventile.

4. Vorrichtung (10) nach Anspruch 3, wobei die Vielzahl von zusammenarbeitet Blättchen (108, 110, 112) oder das erste Exspirationsventil öffnen bei 10 Zentimeter Wasser und 14 Zentimeter Wasser: oder bei 12 Zentimeter Wasser und schließt bei zwischen 6 Zentimeter Wasser und 10 Zentimeter Wasser oder 8 Zentimeter Wasser und der äußere Donut geformt dazwischen öffnet sich der flexible Membranabschnitt (102) oder das zweite Ausatmungsventil 14 Zentimeter Wasser und 18 Zentimeter Wasser oder 16 Zentimeter Wasser und zwischen 10 Zentimeter Wasser und 14 Zentimeter Wasser oder bei 12 Zentimeter Wasser, um dadurch einen positiven Enddruck innerhalb zu halten die Vorrichtung verbraucht während eines Volumenstroms die ausgeatmete Luft schnell ab Rate der ausgeatmeten Luft steigt.

5. Vorrichtung (10) nach Anspruch 2 oder Anspruch 3 oder 4, wie an den Anspruch angehängt 2, wobei das Ausatmungsventil (38) einen positiven Enddruck aufrechterhält und öffnet bei niedrigem Druck, um eine Überdruckbeaufschlagung der Atemwege des Patienten zu verhindern Dadurch wird ein Druck im Luftweg des Patienten zwischen 4 und 14 gehalten Zentimeter Wasser oder 8 Zentimeter Wasser.

6. Vorrichtung (10) nach einem der vorhergehenden Ansprüche, wobei die primäre und Sekundäreinlässe (20, 31) sind an unterschiedlichen Stellen voneinander beabstandet entlang eines einzigen Durchgangs (18) oder entlang verschiedener Durchgänge, oder sich am selben Ort entlang des einzelnen Durchgangs (18) befinden.

7. Vorrichtung (10) nach einem der vorhergehenden Ansprüche, wobei die primäre Einatmungsventil ist ein Membran-Rückschlagventil mit flexiblem Gummimembran über mindestens einer Öffnung im primären Einlass und der positioniert Sekundäres Inspirationsventil ist ein Membran-Rückschlagventil einschließlich eines flexiblen Gummimembran, die über mindestens einer Öffnung im sekundären Einlass positioniert ist.

8. Vorrichtung (10) nach Anspruch 7, bei der das primäre Inspirationsventil ist (28) hat einen niedrigeren Öffnungsdruck im Vergleich zum sekundären Inspirationsventil (30), so dass das primäre Inspirationsventil vor dem sekundären öffnet Einatmungsventil, wobei sauerstoff- oder sauerstoffreiche Luft bevorzugt eingeatmet wird aus dem zusammenlegbaren Reservoirbeutel (24) und wenn besagter zusammenlegbar Reservoirbeutel wird im Wesentlichen durch das sekundäre Inspirationsventil (30) entleert öffnet sich, um den Patienten mit einer Atemluftquelle zu versorgen.

9. Vorrichtung (10) nach Anspruch 8, wobei der primäre und der sekundäre Inspirationsventile (28, 30) sind in einem Kombiventil oder einem Stufeninspirationsventil (76), wobei das kombinierte oder zweistufige Inspirationsventil vorgesehen ist innerhalb eines Gehäuses (78) angeordnet ist, das einen zentralen Rahmen (84) aufweist Öffnungen (86), die sich durch sie hindurch erstrecken, in Fluidverbindung mit der zusammenlegbarer Reservoirbeutel (24) und periphere Öffnungen (88) im Rahmen in Flüssigkeit Kommunikation mit der Umgebungsluft, wobei die primären Inspirationsventile (28) enthält eine scheibenförmige, flexible Membran (80), die an dieser befestigt ist Rahmen (84) und konfiguriert, um die zentralen Öffnungen (86) und die das sekundäre Inspirationsventil (30) umfasst eine ringförmige flexible Membran (82) an dem Rahmen (84) befestigt und konfiguriert ist, um das Gehäuse reversibel abzudichten periphere Öffnungen (88).

10. Vorrichtung (10) nach Anspruch 2 oder einem der Ansprüche 3 bis 9 als an Anspruch 2 angehängt, wobei das Ausatmungsventil (38) primär inspiratorisch ist Das Ventil (28) und das sekundäre Inspirationsventil (30) umfassen jeweils ein Rückschlagventil oder Einwegeventil, ausgewählt aus einer Gruppe mit Membrankontrolle Ventile, Kugelrückschlagventile, Entenschnabelventile, Kippscheibenrückschlagventile, Lift-Check Ventile und Inline-Rückschlagventile.

11. Vorrichtung (10) nach einem der vorhergehenden Ansprüche, wobei die zusammenklappbar sind Der Reservoirbeutel (24) hat ein Volumen zwischen 100 und 600 Milliliter (ml) oder 500 ml. und über dem Primäreinlass (20) unter Verwendung eines Klebstoffs an dem Körper befestigt ist oder Der zusammenlegbare Reservoirbeutel ist an dem Körper heißgeschweißt oder auf andere Weise befestigt.

12. Vorrichtung (10) nach Anspruch 2 oder einem der Ansprüche 3 bis 11 als nach Anspruch 2, wobei sich das erste Inspirationsventil während des Ausatmens öffnet wenn ein Druck innerhalb des zusammenlegbaren Reservoirbeutels (24) eine vorbestimmte Höhe erreicht, wodurch zumindest ein Teil der sauerstoffreichen Luft aus dem Zusammenfall zusammenfällt Der Reservoirbeutel kann in den Durchgang oder die Durchgänge fließen und durch das Ausatmungsventil nach außen, um dadurch das Reißen des zu verhindern faltbarer Reservoirbeutel.

13. Vorrichtung (10) nach Anspruch 11, bei der der zusammenlegbare Reservoirbeutel (24) liefert auditive Nachweise der Atmung, wobei das zusammenklappbare Reservoir Der Beutel gibt beim Befüllen oder Entleeren ein Geräusch über einen Luftdruck aus Geräuschabgabevorrichtung, die aktiviert wird, wenn Luft durch sie strömt, oder das Geräusch wird aufgrund des Materials abgegeben, aus dem sich der zusammenklappbare Reservoirbeutel befindet gebaut.

14. Vorrichtung (10) nach einem der vorhergehenden Ansprüche, wobei die Kupplung (14) konfiguriert ist, um mit einem Filterelement (98) in Eingriff zu treten, das zwischen dem positioniert ist Beatmungsgerät und die Vorrichtung, wobei die Kupplung (14) einstückig mit der ist Körperabschnitt (12) oder einteilig ausgebildet oder die Kupplung damit verbunden ist der Körperteil.

## Revendications

1. Un appareil oxygénant jetable (10) à utiliser sur un patient qui se remet de d'anesthésie ou nécessitant un soutien respiratoire, lorsqu'un appareil respiratoire dispositif est in situ ou doit être utilisé, y compris
une partie du corps (12) ayant un accouplement (14) pour l'attachement à ladite dispositif une extrémité (16) d'un tube endotrachéal ou un tube dudit masque laryngé voies respiratoires, qui s'étend extérieurement à partir d'une voie aérienne dudit patient,
une entrée primaire (20),
une entrée secondaire (31),
une déversoir (22),
un passage (18) ou des passages pour la communication fluide avec les respiratoires dispositif, le passage (18) ou les passages qui s'étendent par ladite portion du corps (12) entre ledit accouplement (14) et l'entrée primaire (20) et l'entrée secondaire (31), et s'étendant à travers ladite partie du corps (12) entre ledit accouplement (14) et la déversoir (22), pour la communication fluide entre eux,
un sac de réservoir pliable (24) attaché sur ledit entrée primaire (20), ledit sac de réservoir pliable en communication fluide avec une source d'oxygène ou air riche en oxygène (25), une vanne expiratoire (38) ou des vannes situées dans ou adjacentes ladite déversoir (22) pour contrôler le passage d'un air expiré de ladite patiente pendant l'expiration, où une pression positive est appliquée aux voies respiratoires de ladite patient pendant l'expiration et le repos,
une vanne inspiratoire primaire (28) située à l'entrée principale (20) ou adjacente, pour contrôler ledit air riche en oxygène ou en oxygène qui coule à travers ledit passage (18) ou des passages de ladite sac de réservoir pliable (24), au moins une période de temps pendant l'inspiration par ledit patient, et une vanne inspiratoire secondaire (30) située dans ou adjacente ladite entrée secondaire (31) dans la communication fluide avec ledit passage (18) ou les passages contrôle de l'entrée d'un air ambiant à travers le passage ou les passages à partir d'un extérieur dudit appareil pendant l'inspiration quand on dit sac de réservoir pliable (24) a été largement vidé.

2. L'appareil (10) selon la revendication 1, dans lequel le dispositif respiratoire est tube endotrachéal ou le masque laryngé des voies respiratoires, dans lequel l'appareil est attaché à une extrémité d'un tube endotrachéal ou d'un tube de ces voies respiratoires du masque laryngé, qui s'étend extérieurement à partir d'une voie aérienne dudit patient, ou le dispositif respiratoire comprend un masque qu'il a attaché sur une bouche et un nez dudit patient, dans lequel l'appareil est attaché à ledit masque ou un tube relié à ce masque facial, ou le dispositif respiratoire comprend un nasal dispositif de soutien respiratoire des voies respiratoires.

3. L'appareil (10) selon la revendication 1 ou 2, dans lequel la vanne expiratoire (38) située dans la déversoir (22) ou adjacente, est un clapet de contrôle en ligne, ou valve expiratoire comprend une vanne expiratoire à deux étages comprenant une portion centrale flexible (100), et une diaphragme extérieure en forme de Donut plus grande portion (102), la portion centrale flexible (100), y compris une pluralité de feuilles coopératifs (108, 1 10, 112), auquel cas la portion centrale flexible, c'est-à-dire la première valve expiratoire, s'ouvre en premier, et l'air est de préférence expulsé, à partir de l'intérieur du passage (18) ou passages pendant l'expiration, et si ledit patient tousse la portion externe de diaphragme flexible en forme de beignet (102), c'est-à-dire que la deuxième valve expiratoire s'ouvre pour dissiper rapidement l'air expiré, c'est-à-dire que la vanne expiratoire (38) comprend première et deuxième valve expiratoires.

4. L'appareil (10) selon la revendication 3, dans lequel la pluralité de feuilles coopératifs (108, 110, 112) ou la première valve expiratoire s'ouvrent entre 10 centimètres d'eau (cmH₂O) et 14 cmH₂O: ou à 12 cmH₂O, et ferme à entre 6 cmH₂O et 10 cmH₂O, ou 8 cmH₂O, et le diaphragme extérieure en forme de Donut (102) ou la seconde valve expiratoire s'ouvre entre 14 cmH₂O et 18 cmH₂O, ou 16 cmH₂O, et proche entre 10 cmH₂O et 14 cmH₂O, ou à 12 cmH₂O, pour maintenir ainsi une pression finale positive dans l'appareil tout en dissipant rapidement l'air expiré lorsqu'un flux volumétrique le taux d'air expiré augmente.

5. Appareil (10) selon la revendication 2 ou les revendications 3 ou 4, annexé à la revendication 2, dans laquelle la vanne expiratoire (38) maintient une pression finale positive et s'ouvre à basse pression pour empêcher la surpression des voies respiratoires du patient, pour maintenir ainsi une pression dans les voies respiratoires du patient comprise entre 4 et 14 centimètres d'eau (cmH₂O) ou 8 cmH₂O.

6. Appareil (10) selon l'une quelconque des revendications précédentes, dans lequel les primaire et les entrées secondaires (20, 31) sont espacées les unes des autres à des emplacements différents le long d'un passage unique (18), ou sont situés le long de différents passages, ou sont situés au même endroit le long dudit passage unique (18).

7. Appareil (10) selon l'une quelconque des revendications précédentes, dans lequel le primaire la valve inspiratoire est un clapet anti-retour à membrane comprenant un caoutchouc flexible diaphragme positionné sur au moins une ouverture de l'entrée principale et le la valve inspiratoire secondaire est un clapet anti-retour à membrane comprenant un caoutchouc flexible diaphragme positionné sur au moins une ouverture de l'entrée secondaire.

8. Appareil (10) selon la revendication 7, dans lequel la vanne inspiratoire primaire (28) a une pression d'ouverture inférieure à celle de la vanne inspiratoire secondaire (30), de telle sorte que la vanne inspiratoire primaire s'ouvre avant le secondaire vanne inspiratoire, dans laquelle l'oxygène ou l'air riche en oxygène est inhalé préférentiellement depuis l'intérieur du sac de réservoir pliable (24) et lorsque ledit sac réservoir pliable est pratiquement vidé de la vanne inspiratoire secondaire (30) s'ouvre pour fournir au patient une source d'air respirable.

9. Appareil (10) selon la revendication 8, dans lequel lesdites primaire et secondaire les vannes inspiratoires (28, 30) sont incorporées dans une valve combinée ou dans un valve inspiratoire à deux étages (76), ladite valve combinée ou valve inspiratoire à deux étages se trouvant dans un boîtier (78) ayant un cadre (84) comprenant des éléments centraux des ouvertures (86) s'étendant à travers celles-ci, en communication fluide avec le sac de réservoir pliable (24) et ouvertures périphériques (88) dans le cadre en fluide communication avec l'air ambiant, les vannes inspiratoires primaires (28) comprend un diaphragme flexible en forme de disque (80) fixé à ladite cadre (84) et configuré pour sceller de manière réversible les ouvertures centrales (86), et la vanne inspiratoire secondaire (30) comprend une membrane flexible en forme d'anneau (82) étant fixé audit cadre (84) et configuré pour sceller de manière réversible le ouvertures périphériques (88).

10. Appareil (10) selon la revendication 2 ou l'une quelconque des revendications 3 à 9, tel que annexée à la revendication 2, dans laquelle la vanne expiratoire (38), vanne inspiratoire primaire (28) et la vanne inspiratoire secondaire (30) comprennent chacune une valve anti-retour ou une valve à sens unique, étant choisi dans un groupe contenant le contrôle de la membrane vannes, clapets anti-retour à bille, vannes à bec de canard, clapets anti-retour à disque basculant, levée-contrôle vannes et clapets anti-retour en ligne.

11. Appareil (10) selon l'une quelconque des revendications précédentes, dans lequel le sac de réservoir pliable (24) a un volume compris entre 100 et 600 millilitres (mL) ou 500 mL, et est fixé au corps par-dessus l'entrée primaire (20) à l'aide d'un adhésif, ou le sac de réservoir pliable est thermosoudé ou autrement fixé au corps.

12. Appareil (10) selon la revendication 2 ou l'une quelconque des revendications 3 à 11, tel que annexé à la revendication 2, dans lequel la première vanne inspiratoire s'ouvre pendant l'expiration si une pression à l'intérieur de la sac de réservoir pliable (24) atteint un niveau prédéterminé, moyennant quoi au moins une partie de l'air riche en oxygène provenant de l'intérieur du sac pliable peut sortir dans le passage ou les passages et à travers la valve expiratoire, pour empêcher ainsi la rupture de la sac de réservoir pliable.

13. Appareil (10) selon la revendication 11, dans lequel le sac de réservoir pliable (24) fournit des preuves auditives de la respiration, dans lesquelles le sac de réservoir pliable émet un bruit lorsqu'il est rempli ou vidé, par voie de pneumatique dispositif émettant du bruit qui est activé lorsque de l'air le traverse, ou bruit est émis en raison du matériau à partir duquel le sac de réservoir pliable est construit.

14. Appareil (10) selon l'une quelconque des revendications précédentes, dans lequel l'accouplement (14) étant configuré pour engager un élément filtrant (98) positionné entre le appareil respiratoire et ledit appareil, l'accouplement (14) faisant partie intégrante du partie du corps (12), ou de construction unitaire, ou l'accouplement étant connecté à la partie du corps.
